# EUROPÄISCHE PATENTSCHRIFT

(11) **EP 2 421 522 B1**
(45) Veröffentlichungstag und Bekanntmachung des Hinweises auf die Patenterteilung: **03.04.2013**
(21) Anmeldenummer: 10714862.9
(22) Anmeldetag: 19.04.2010
(51) Int. Cl.: A61K 31/167, A61K 47/10, A61K 9/00, A61P 29/02

(54) **PARACETAMOL ZUR PARENTERALEN VERABREICHUNG**
PARACETAMOL FOR PARENTERAL ADMINISTRATION
PARACÉTAMOL DESTINÉ À L'ADMINISTRATION PARENTÉRALE

(30) Priorität: 22.04.2009 EP 09005630
(43) Veröffentlichungstag der Anmeldung: 29.02.2012
(73) Patentinhaber: Fresenius Kabi Deutschland GmbH, 61352 Bad Homburg (DE)
(72) Erfinder: DASBERG, David, 61348 Bad Homburg (DE); ACHLEITNER, Georg, A-8010 Graz (AT); AICHHOLZER, Christine, A-8010 Graz (AT)
(86) Internationale Anmeldenummer: PCT/EP2010/002368
(87) Internationale Veröffentlichungsnummer: WO 2010/121762

(56) Entgegenhaltungen:
- WO-A-00/07588
- WO-A-01/08662
- WO-A-2008/007150
- US-A1- 2005 203 175
- DATABASE WPI Week 199444 Thomson Scientific, London, GB; AN 1994-355463 XP002030817 & KR 930 011 994 B1 (DAEKWANG PHARM CO) 23. Dezember 1993 (1993-12-23)

## Beschreibung

Die Erfindung betrifft pharmazeutische Formulierungen des Wirkstoffs Paracetamol (Acetaminophen), welche sich zur parenteralen Verabreichung, insbesondere Infusion eignen.

Paracetamol ist ein sehr breit eingesetzter Wirkstoff mit exzellenter Verträglichkeit (vgl. z.B. G.G. Graham et al., Drug Safety, 2005, 28(3), 227-40). Paracetamol ist in zahlreichen Arzneiformen kommerziell erhältlich, insbesondere als orale Darreichungsform. In bestimmten Fällen, beispielsweise im Rahmen einer intensivmedizinischen Behandlung oder wenn eine orale Verabreichung aus bestimmten Gründen nicht möglich ist, ist eine parenterale Verabreichung von Paracetamol wünschenswert.

Das Arzneibuch versteht unter Parenteralia sterile Zubereitungen, die zur Injektion, Infusion oder Implantation bestimmt sind. Parenteralia müssen grundsätzlich mit besonderer Sorgfalt hergestellt werden, um Reizlosigkeit zu garantieren und mikrobielle und partikuläre Verunreinigungen zu vermeiden. Als Hilfsstoffe kommen vor allem Stoffe zur Verbesserung der Löslichkeit, Stoffe zur Isotonisierung, Puffer, Antioxidantien, Chelatbildner, Konservierungsmittel, Emulgatoren und Hilfsstoffe zur Wirkungsverlängerung in Betracht.

Wässrige Parenteralia müssen der Blut- bzw. Gewebsflüssigkeit isoosmotisch bzw. annähernd isoosmotisch sein. Bei stärker hypo- oder hyperosmotischen Abweichungen kommt es sonst zu Erythrozytenschädigung bzw. Gewebereizungen. Bei intravenöser Gabe stärker hypoosmotischer Lösungen tritt Hämolyse, bei Zufuhr größerer Mengen hyperosmotischer Lösungen tritt Plasmolyse ein.

Auch der pH-Wert wässriger Parenteralia spielt eine wichtige Rolle. Das Blutserum verfügt über die vier Puffersysteme Kohlensäure/Hydrogencarbonat, Plasmaproteine, primäres/sekundäres Phosphat und Hämoglobin/Oxyhämoglobin. Der pH-Wert des Bluts liegt zwischen 7,30 und 7,45. Eine Angleichung des pH-Werts von Infusionslösungen an den physiologischen pH-Bereich (Isohydrie) ist aus Stabilitätsgründen häufig nicht möglich. Es erfolgt dann lediglich eine bestmögliche Angleichung an den physiologischen pH-Bereich (Euhydrie). Der Toleranzbereich für Infusionslösungen liegt im Allgemeinen zwischen pH 3,0 und 10,5. Je nach Abweichung des tatsächlichen pH-Werts vom physiologischen pH-Bereich ist dann eine ausreichend langsame Infusion erforderlich, um den Puffersystemen des Bluts eine Angleichung an den physiologischen pH-Bereich zu ermöglichen.

Eine Pufferung von Infusionslöungen, z.B. mit Acetat-, Phosphat- oder Citratpuffern, hat den Nachteil, dass sie die natürliche pH-Stabilisierung des Bluts überlagert. Damit die natürliche pH-Stabilisierung des Bluts erhalten bleibt, sollte eine Pufferung von Infusionsarzneimitteln daher möglichst nicht erfolgen. Die Einstellung des pH-Werts mit starken Säuren bzw. Basen (z.B. HCl bzw. NaOH) hat hingegen keine Pufferwirkung zur Folge und ist daher weniger bedenklich.

Infusionslösungen von Paracetamol sind im Stand der Technik bekannt.

In der Bundesrepublik Deutschland wird eine Infusionslösung von Paracetamol unter der Bezeichnung Perfalgan^{®} vermarktet. Die Infusionslösung ist indiziert für eine Kurzzeitbehandlung von mäßig starken Schmerzen, besonders nach Operationen, und für die Kurzzeitbehandlung von Fieber, wenn die intravenöse Anwendung aufgrund einer dringend erforderlichen Schmerz- oder Fieberbehandlung klinisch gerechtfertigt ist bzw. wenn andere Arten der Anwendung nicht möglich sind. Die Art der Anwendung erfolgt als 15-minütige Infusion. Neben Paracetamol enthält die Infusionslösung als sonstige Bestandteile Cysteinhydrochlorid-Monohydrat, Natriummonohydrogenphosphat-Dihydrat, Salzsäure, Mannitol, Natriumhydroxid und Wasser für Injektionszwecke. Der Gehalt an Natrium wird mit 0,04 mg/ml angegeben. Die Haltbarkeit wird mit 2 Jahren angegeben, wobei eine Lagerung nicht über 30 °C und nicht im Kühlschrank erfolgen sollte.

EP-A 916 347 offenbart gepufferte Paracetamol-Injektionsformen auf Basis organischer Lösungsmittel, insbesondere Ethanol und Benzylalkohol. Als Stabilisatoren werden Chelatbildner und Antioxidanzien zugesetzt.

US-A 2005/0203175 offenbart gepufferte Zusammensetzungen zur parenteralen Verabreichung von Paracetamol in Kombination mit Lidocain HCl, welche u.a. organische Lösungsmittel, Chelatbildner und Antioxidanzien enthalten.

WO 02/072080 betrifft gepufferte wässrige Lösungen von Paracetamol und Antioxidantien ausgewählt aus der Gruppe bestehend aus Ascorbinsäure, N-Acetyl-L-Cystein und anderer SH-Gruppen-haltiger Stabilisatoren. Die Lösungen sind mit NaCl isotonisiert.

US 6,028,222 offenbart gepufferte wässrige Lösungen von Paracetamol, welche einen Radikalfänger oder Radikalantagonisten enthalten.

WO 03/033026 betrifft wässrige Lösungen von Pracetamol, welche Propylenglykol und Citratpuffer enthalten und durch eine bestimmte Wärmebehandlung erhältlich sind.

EP-A 1 889 607 offenbart gepufferte wässrige Lösungen von Pracetamol, welche Glucose, Fructose oder Gluconat und Formaldehyd-Sulfoxylat, Natriumsilfit oder Natriumdithionit enthalten.

EP-A 1 752 139 betrifft wässrige Lösungen von Paracetamol und Antioxidantien ausgewählt aus der Gruppe bestehend aus Ascorbinsäure, N-Acetyl-L-Cystein und anderer SH-Gruppen-haltiger Stabilisatoren. Die Lösungen sind mit NaCl isotonisiert und weisen einen Sauerstoffgehalt von weniger als 1 mg/l auf.

US 6,992,218 und FR-A 2 809 619 betreffen Verfahren zur Herstellung gepufferter, wässriger Lösungen von Paracetamol mit einem Sauerstoffgehalt von weniger als 2 ppm.

US 2006/0084703 offenbart wässrige Formulierungen von Paracetamol, welche Puffer, Isotonisierungsmittel und ein Paracetamol-Dimer enthalten.

US 2006/0292214 betrifft Zusammensetzungen, welche Paracetamol in nanopartikulärer Form enthalten.

KR 930 011 994 offenbart gemäß Zusammenfassung Zusammensetzungen u.a. zur parenteralen Verabreichung von Paracetamol, bei denen der Wirkstoff in Polyethylenglykol und Propylenglykol gelöst ist. Die Zusammensetzungen eignen sich zur Herstellung von Tabletten, Kapseln, Sirupen, Suppositorien und Injektionszubereitungen. Hinweise auf wässrige Zusammensetzungen können der Zusammenfassung jedoch nicht entnommen werden, insbesondere auch keine Infusionslösungen.

WO 00/07588 betrifft eine im Wesentlichen wasserfreie Paracetamolzusammensetzung, welche Polyethylengykol und Alkohol enthält und vor der Anwendung mit Wasser zu einer injezierbaren Lösung verdünnt wird.

US 2005/203175 offenbart eine Paracetamolzusammensetzung, welche Chelatbildner, Antioxidationsmittel und Puffer enthält.

WO 01/08662 betrifft eine im Wesentlichen wasserfreie Paracetamolzusammensetzung zur nasalen Verabreichung.

WO 2008/007150 offenbart eine Paracetamolnanodispersion zur oralen Verabreichung.

Die aus dem Stand der Technik bekannten pharmazeutischen Zusammensetzungen zur parenteralen Verabreichung von Paracetamol sind jedoch nicht in jeglicher Hinsicht zufriedenstellend. Paracetamol ist vergleichsweise schlecht löslich und oxidationsempfindlich, weshalb üblicherweise entsprechende Maßnahmen getroffen werden, um eine ausreichende Lagerstabilität der Zusammensetzungen zu gewährleisten.

So sind die pharmazeutischen Zusammensetzungen zur parenteralen Verabreichung von Paracetamol üblicherweise gepuffert, so dass die natürliche Pufferwirkung des Bluts durch die Puffer überlagert wird und ggf. eine nur vergleichsweise langsame Infusion möglich ist. Im Falle von Phosphat-Puffern können sich insbesondere mit zweiwertigen Metallkationen (Ca²⁺, Mg²⁺) unlösliche Komplexe bilden. Dies kann sich nicht nur bei Patienten mit entsprechenden Mangelerscheinungen negativ auswirken, sondern verkompliziert auch die Coinfusion mit entsprechenden Elektrolytlösungen, welche u.U. indiziert sein kann.

Ferner enthalten viele der bekannten pharmazeutischen Zusammensetzungen vergleichsweise hohe Elektrolytkonzentrationen, insbesondere auch Natriumionen, was zu einer osmotischen Verschiebung von Wasser aus den Zellen in das Interstitium führen kann.

Darüber hinaus enthalten die pharmazeutischen Zusammensetzungen zur parenteralen Verabreichung von Paracetamol üblicherweise eine Vielzahl unterschiedlicher Inhaltsstoffe, was u.a. aus ökonomischer Sicht von Nachteil ist. Wegen der besonderen Anforderungen an Parenteralia müssen besondere Reinheitskriterien eingehalten und regelmäßig analytisch überwacht werden. So enthalten die bekannten pharmazeutischen Zusammensetzungen zur parenteralen Verabreichung von Paracetamol üblicherweise bestimmte Antioxidanzien, welche Unverträglichkeiten und Nebenwirkungen hervorrufen können. Wird auf solche Antioxidantien verzichtet, so resultiert dies üblicherweise in einer geringeren Lagerstabilität.

Andere pharmazeutische Zusammensetzungen zur parenteralen Verabreichung von Parcetamol werden wasserfrei bereitgestellt, insbesondere in Form alkoholischer Lösungen, welche unmittelbar vor der Verabreichung verdünnt werden müssen, beispielsweise mit Wasser. Diese Zusammensetzungen sind also als solche noch nicht gebrauchsfertig, sondern erfordern besondere, relativ umständliche Vorbereitungsmaßnahmen, ehe sie angewendet werden können. Die daraus resultierenden alkoholisch-wässrigen Zusammensetzungen weisen nicht zuletzt wegen des Alkoholgehalts erhebliche Nachteile auf und sind beispielsweise zur Infusion ungeeignet. Auch bringen die Vorbereitungsmaßnahmen wie Verdünnungen immer die Gefahr mit sich, Kontaminationen einzuschleppen, was unter Sterilitätskriterien bedenklich ist.

Der Erfindung liegt die Aufgabe zugrunde, pharmazeutische Zusammensetzungen zur parenteralen Verabreichung von Paracetamol bereitzustellen, welche Vorteile gegenüber den Zusammensetzungen des Standes der Technik aufweisen.

Diese Aufgabe wird durch den Gegenstand der Patentansprüche gelöst.

Es wurde überraschend gefunden, dass Paracetamol gegenüber oxidativem Abbau stabilisiert werden kann, wenn die Elektrolytkonzentration gering gehalten wird. Der Zusatz von Elektrolyten führt zu einer Destabilislerung.

Legt man als Maß für den Gehalt an Elektrolyten die elektrische Leitfähigkeit zugrunde, so nimmt die Lagerstabilität mit steigender elektrischer Leitfähigkeit ab. Dadurch wird es möglich, Zusammensetzungen zur parenteralen Verabreichung von Paracetamol bereitzustellen, insbesondere Infusionslösungen, welche mit einem Minimum an Inhaltsstoffen auskommen und dennoch eine hinreichende Lagerstabilität aufweisen.

Werden als Inhaltsstoffe durchweg nicht-ionische oder zwitterionische Verbindungen, welche nach außen elektrisch neutral sind, zugesetzt, so erhöhen diese die elektrische Leitfähigkeit der Zusammensetzung praktisch nicht, wodurch die hohe Lagerstabllität der Zusammensetzung erhalten bleibt und ggf., je nach Art des Inhaltsstoffs, weiter verbessert werden kann.

Die Erfindung betrifft eine wässrige pharmazeutische Zusammensetzung zur parenteralen Verabreichung, vorzugsweise in Form einer Infusionslösung, welche Paracetamol enthält und eine elektrische Leitfähigkeit von höchstens 200 µS cm⁻¹ aufweist, wobei die Zusammensetzung keine C₁-C₆-Alkanole und kein Polyethylenglykol enthält.

Die erfindungsgemäße Zusammensetzung, vorzugsweise Infusionslösung, ist wässrig. Da sie zur parenteralen Verabreichung vorgesehen ist, enthält sie bevorzugt Wasser für Injektionszwecke (Ph. Eur.). Bevorzugt ist Wasser für Injektionszwecke der einzige flüssige Bestandteil der erfindungsgemäßen Zusammensetzung. So enthält die erfindungsgemäße Zusammensetzung vorzugsweise keine organischen Lösungsmittel. Hierunter fallen alle dem Fachmann bekannten, im Wesentlichen niedermolekularen organischen Verbindungen, die ausschließlich zum Zwecke der Erhöhung der Löslichkeit des Paracetamols in Wasser eingesetzt werden. Hierzu gehören vor allem Alkohole, insbesondere C₁-C₆-Alkanole wie Ethanol, Propylenglykol, Glycerin, Benzylalkohol und andere niedermolekulare organische Verbindungen, die Hydroxylgruppen enthalten. Weitere Vertreter sind höhermolekulare Substanzen, insbesondere Polyethylenglykol, Polypropylenglykol und deren Mischungen oder Copolymere. Besonders bevorzugt enthält die erfindungsgemäße Zusammensetzung keine organischen Lösungsmittel ausgewählt aus der Gruppe bestehend aus C₁-C₆-Alkanolen, Propylenglykol und Polyethylenglykol (PEG).

Infusionslösungen und Injektionslösungen sind grundsätzlich voneinander zu unterscheiden.

Bei der Injektion handelt es sich um das Einspritzen von sterilen Arzneimitteln in den Körper mit einer Spritze und einer Hohlnadel. Das Volumen einer Injektion beträgt üblicherweise 0,1 bis 20 ml. Im Gegensatz zur Infusion wird das Arzneimittel innerhalb von Sekunden bis zu wenigen Minuten verabreicht, wobei manueller Druck auf die Spritze ausgeübt wird. Injektionen erfolgen häufig nicht intravenös, sondern z.B. subcutan oder intramuskulär. Dabei soll entweder eine lokale Wirkung erreicht werden oder aber für eine systemische Wirkung wird lokal eine Art Wirkstoffdepot ausgebildet, von wo aus der Wirkstoff erst nach und nach an den Kreislauf abgegeben wird. Werden Medikamente intravenös injiziert, ist hohe Vorsicht geboten. Es ist sehr wichtig, dass die Geschwindigkeit bei der Verabreichung (zw. 1-3 ml pro Minute) genau eingehalten wird. Es dürfen maximal 5 ml auf diese Weise gespritzt werden. Ist die erforderliche Dosis höher, muss das Medikament in eine Trägerlösung injiziert und über eine Kurzinfusion gegeben werden. Der grundsätzliche Unterschied zur Infusion wird auch daran deutlich, dass bei Injektionen üblicherweise darauf zu achten ist, dass größere Gefäße gerade nicht mit der Nadel getroffen werden, da sonst eine unbeabsichtigte intravenöse oder intrarterielle Verabreichung erreicht würde, welche beispielsweise einen anaphälaktischen Schock auslösen kann.

Bei der Infusion handelt es sich üblicherweise um ein langsames, meist tropfenweises Einfließen größerer (arzneimittelhaltiger) Flüssigkeitsmengen in den Körper, wobei man je nach Zeitdauer der Infusion zwischen Dauerinfusionen (mehrere Stunden, oft auch 24 Stunden ununterbrochen) und Kurzzeitinfusionen (weniger als 3 Stunden, oft 15-30 Minuten) unterscheiden kann. Das Einfließen erfolgt üblicherweise aufgrund des hydrostatischen Drucks der Flüssigkeitssäule im Zulauf, im Unterschied zur Injektion nicht jedoch durch aktives Druckausüben. Infusionen erfolgen häufig intravenös. Die Zusammensetzung von Infusionslösungen unterscheidet sich daher meist grundlegend von der Zusammensetzung von Injektionslösungen, insbesondere auch im Hinblick auf die Wirkstoffkonzentration. Bevorzugt liegen in der erfindungsgemäßen Zusammensetzung, vorzugsweise Infusionslösung, alle Inhaltsstoffe vollständig gelöst vor, d.h. es handelt sich bevorzugt um keine Dispersion, weder um eine Emulsion noch um eine Suspension. Die erfindungsgemäße Zusammensetzung ist vorzugsweise partikel- und verfärbungsfrei. Insbesondere enthält die erfindungsgemäße Zusammensetzung bevorzugt auch keine Nanopartikel.

Die erfindungsgemäße Zusammensetzung, vorzugsweise Infusionslösung, enthält Paracetamol (Acetaminophen). Das Paracetamol liegt vorzugsweise in vollständig gelöster Form vor. Die Konzentration des Paracetamols in der erfindungsgemäßen Zusammensetzung liegt vorzugsweise unterhalb seiner Sättigungskonzentration, besonders bevorzugt wenigstens 95% unterhalb seiner Sättigungskonzentration bei Raumtemperatur. In einer bevorzugten Ausführungsform liegt die Konzentration des Paracetamols im Bereich von 10,0 ± 7,5 gl⁻¹, 10,0 ± 6,0 gl⁻¹, 10,0 ± 5,0 gl⁻¹, 10,0 ± 4,0 gl⁻¹, 10,0 ± 3,0 g l⁻¹ oder 10,0 ± 2,5 g l⁻¹; bevorzugter 10,0 ± 2,0 g l⁻¹, noch bevorzugter 10,0 ± 1,5 g l⁻¹, am bevorzugtesten 10,0 ± 1,0 g l⁻¹ und insbesondere 10,0 ± 0,5 g l⁻¹, bezogen auf die Zusammensetzung.

In einer bevorzugten Ausführungsform beträgt der Gehalt an Paracetamol entweder weniger als 1,2 Gew.-% oder mehr als 1,3 Gew.-%, bezogen auf die Zusammensetzung.

Die erfindungsgemäße Zusammensetzung, vorzugsweise Infusionslösung, kann neben Paracetamol weitere Wirkstoffe enthalten. Vorzugsweise enthält die erfindungsgemäße Zusammensetzung jedoch Paracetamol als einzigen Wirkstoff.

Die erfindungsgemäße Zusammensetzung, vorzugsweise Infusionslösung, weist eine elektrische Leitfähigkeit von höchstens 200 µS cm⁻¹ auf. Die Messung der elektrischen Leitfähigkeit wässriger Lösungen ist dem Fachmann bekannt und geeignete Messgeräte sind kommerziell erhältlich. Vorzugsweise wird die elektrische Leitfähigkeit bei Raumtemperatur gemessen. Bevorzugt beträgt die elektrische Leitfähigkeit der erfindungsgemäßen Zusammensetzung höchstens 190 µS cm⁻¹, höchstens 180 µS cm⁻¹, höchstens 170 µS cm⁻¹, höchstens 160 µS cm⁻¹, höchstens 150 µS cm⁻¹, höchstens 140 µS cm⁻¹, höchstens 130 µS cm⁻¹, höchstens 120 µS cm⁻¹ oder höchstens 110 µS cm⁻¹; bevorzugter höchstens 100 µS cm⁻¹ höchstens 90 µS cm⁻¹, höchstens 80 µS cm⁻¹, höchstens 70 µS cm⁻¹, oder höchstens 60 µS cm⁻¹; noch bevorzugter höchstens 50 µS cm⁻¹, höchstens 40 µS cm⁻¹, oder höchstens 30 µS cm⁻¹; am bevorzugtesten höchstens 25 µS cm⁻¹, höchstens 20 µS cm⁻¹ oder höchstens 15 µS cm⁻¹; und insbesondere höchstens 12,5 µS cm⁻¹, höchstens 10 µS cm⁻¹ oder höchstens 7,5 µS cm⁻¹.

Damit zeichnet sich die erfindungsgemäße Zusammensetzung, vorzugsweise Infusionslösung, durch eine vergleichsweise geringe elektrische Leitfähigkeit aus. So liegt im Vergleich die elektrische Leitfähigkeit einer isotonen Kochsalzlösung (0,9 Gew.-% NaCl) bei mehr als 7500 µS cm⁻¹. Die elektrische Leitfähigkeit wässriger Zusammensetzungen wird wesentlich durch Ionen beeinflusst. Sie kann auf Grundlage des Quadratwurzelgesetztes nach Kohlrausch oder der Debye-Hückel-Onsager Theorie vorausgesagt werden. Wie im experimentellen Teil näher erläutert, trägt Paracetamol selbst praktisch nicht zur elektrischen Leitfähigkeit bei (10 g Paracetamol in 1000 ml Wasser: ca. 4 µS/cm). Ein Zusatz von 100 mg NaCl zu dieser Wirkstofflösung (0,01 Gew.-% NaCl) führt jedoch bereits zu einem Anstieg der elektrischen Leitfähigkeit auf ca. 200 µS/cm.

Im Zusammenhang mit pharmazeutischen Zusammensetzungen zur parenteralen Verabreichung, vorzugsweise Infusionslösungen, haben insbesondere Elektrolyte und Puffer einen Einfluss auf die elektrische Leitfähigkeit. Demnach enthält die erfindungsgemäße Zusammensetzung, wenn überhaupt, allenfalls eine vergleichsweise geringe Menge an Elektrolyten und/oder Puffersubstanzen.

In einer bevorzugten Ausführungsform enthält die erfindungsgemäße Zusammensetzung, vorzugsweise Infusionslösung, praktisch keine dreiwertigen Elektrolyte, z.B. PO₄³⁻ und HOC(CO₂⁻)₃. In einer anderen bevorzugten Ausführungsform enthält die erfindungsgemäße Zusammensetzung praktisch keine zweiwertigen Elektrolyte, z.B. Ca²⁺, Mg²⁺, HPO₄²⁻ und HOC(CO₂⁻)₂CO₂H. In einer weiteren bevorzugten Ausführungsform enthält die erfindungsgemäße Zusammensetzung praktisch keine einwertigen Elektrolyte, z.B. Na⁺, K⁺, NH₄⁺, Cl⁻, CH₃CO₂⁻, H₂PO₄⁻ und HOCCO₂⁻(CO₂H)₂.

In einer bevorzugten Ausführungsform weist die erfindungsgemäße Zusammensetzung, vorzugsweise Infusionslösung, eine Pufferkapazität β von höchstens 5 mmol l⁻¹ pH⁻¹ auf. Die Definition und die Bestimmung der Pufferkapazität β sind dem Fachmann bekannt. Im Allgemeinen ist die Pufferkapazität diejenige Stoffmenge eines starken Proteolyten (Säure oder Base), welche erforderlich ist, um den pH-Wert der Zusammensetzung um eine Einheit zu verändern. Bevorzugt erfolgt die Messung der Pufferkapazität bei Raumtemperatur.

Bevorzugt weist die erfindungsgemäße Zusammensetzung, vorzugsweise Infusionslösung, eine Pufferkapazität β von höchstens 4,5 mmol l⁻¹ pH⁻¹, höchstens 4,0 mmol l⁻¹ pH⁻¹, höchstens 3,5 mmol l⁻¹ pH⁻¹, höchstens 3,0 mmol l⁻¹ pH⁻¹, höchstens 2,5 mmol l⁻¹ pH⁻¹, höchstens 2,0 mmol l⁻¹ pH⁻¹, höchstens 1,5 mmol l⁻¹ pH⁻¹, oder höchstens 1,0 mmol l⁻¹ pH⁻¹; bevorzugter höchstens 0,9 mmol l⁻¹ pH⁻¹, höchstens 0,8 mmol l⁻¹ pH⁻¹, höchstens 0,7 mmol l⁻¹ pH⁻¹, höchstens 0,6 mmol l⁻¹ pH⁻¹, höchstens 0,5 mmol l⁻¹ pH⁻¹, höchstens 0,4 mmol l⁻¹ pH⁻¹, höchstens 0,3 mmol l⁻¹ pH⁻¹, höchstens 0,2 mmol l⁻¹ pH⁻¹, oder höchstens 0,1 mmol l⁻¹ pH⁻¹; noch bevorzugter höchstens 0,09 mmol l⁻¹ pH⁻¹, höchstens 0,08 mmol l⁻¹ pH⁻¹, höchstens 0,07 mmol l⁻¹ pH⁻¹, höchstens 0,06 mmol l⁻¹ pH⁻¹, höchstens 0,05 mmol l⁻¹ pH⁻¹, höchstens 0,04 mmol l⁻¹ pH⁻¹ , höchstens 0,03 mmol l⁻¹ pH⁻¹, höchstens 0,02 mmol l⁻¹ pH⁻¹ oder höchstens 0,01 mmol l⁻¹ pH⁻¹; am bevorzugtesten höchstens 0,009 mmol l⁻¹ pH⁻¹, höchstens 0,008 mmol l⁻¹ pH⁻¹, höchstens 0,007 mmol l⁻¹ pH⁻¹, höchstens 0,006 mmol l⁻¹ pH⁻¹, oder höchstens 0,005 mmol l⁻¹ pH⁻¹ auf; insbesondere bevorzugt ist die erfindungsgemäße Zusammensetzung praktisch ungepuffert.

Bevorzugt weist die erfindungsgemäße Zusammensetzung, vorzugsweise Infusionslösung, einen pH-Wert im Bereich von 5,0 bis 7,5 auf. In einer bevorzugten Ausführungsform weist die erfindungsgemäße Zusammensetzung einen pH-Wert im Bereich von 5,5±0,5, bevorzugter 5,5±0,4, noch bevorzugter 5,5±0,3, am bevorzugtesten 5,5±0,2 und insbesondere 5,5±0,1 auf. In einer anderen bevorzugten Ausführungsform weist die erfindungsgemäße Zusammensetzung einen pH-Wert im Bereich von 6,0±0,5, bevorzugter 6,0±0,4, noch bevorzugter 6,0±0,3, am bevorzugtesten 6,0±0,2 und insbesondere 6,0±0,1 auf. In einer weiteren bevorzugten Ausführungsform weist die erfindungsgemäße Zusammensetzung einen pH-Wert im Bereich von 6,5±0,5, bevorzugter 6,5±0,4, noch bevorzugter 6,5±0,3, am bevorzugtesten 6,5±0,2 und insbesondere 6,5±0,1 auf. In einer anderen bevorzugten Ausführungsform weist die erfindungsgemäße Zusammensetzung einen pH-Wert im Bereich von 7,0±0,5, bevorzugter 7,0±0,4, noch bevorzugter 7,0±0,3, am bevorzugtesten 7,0±0,2 und insbesondere 7,0±0,1 auf.

In einer besonders bevorzugten Ausführungsform ist der pH-Wert der erfindungsgemäßen Zusammensetzung, vorzugsweise Infusionslösung, nativ, d.h. er wird durch die Inhaltsstoffe festgelegt und weder durch den Zusatz von Puffer noch durch den Zusatz von starker Säure bzw. Base beeinflusst.

Bevorzugt enthält die erfindungsgemäße Zusammensetzung, vorzugsweise Infusionslösung, ein oder mehrere nicht-ionische Isotonisierungsmittel. Geeignete nicht-ionische Isotonisierungsmittel sind dem Fachmann bekannt, insbesondere Glucose, Fructose und Mannit. Bevorzugt handelt es sich bei dem nicht-ionischen Isotonisierungsmittel um einen Zuckeralkohol, insbesondere um Mannitol (Mannit).

Die Konzentration des nicht-ionischen Isotonisierungsmittels, vorzugsweise Mannitols, liegt bevorzugt im Bereich von 35±25 gl⁻¹, bevorzugter 35±20 gl⁻¹, noch bevorzugter 35±15 gl⁻¹, am bevorzugtesten 35±10 gl⁻¹, und insbesondere 35±5 gl⁻¹, bezogen auf die Zusammensetzung. In einer bevorzugten Ausführungsform beträgt der absolute Gehalt an Mannitol entweder weniger als 0,91 Gew.-% oder mehr als 1,17 Gew.-%, bezogen auf die Zusammensetzung, vorzugsweise Infusionslösung.

Bevorzugt ist der Gewichtsanteil des nicht-ionischen Isotonisierungsmittels, vorzugsweise Mannitols, größer als der Gewichtsanteil des Paracetamols in der erfindungsgemäßen Zusammensetzung, vorzugsweise Infusionslösung. Vorzugsweise ist das relative Gewichtsverhältnis nicht-ionisches Isotonisierungsmittel : Paracetamol > 1:1, bevorzugter > 1,5:1, noch bevorzugter > 2:1, am bevorzugtesten > 2,5:1 und insbesondere > 3:1 oder > 3,5:1. In einer bevorzugten Ausführungsform ist das relative Gewichtsverhältnis von Paracetamol zu Mannitol entweder größer als 1:0,7 oder kleiner als 1:1.

In einer bevorzugten Ausführungsform enthält die erfindungsgemäße Zusammensetzung, vorzugsweise Infusionslösung, Cystein, ggf. zusätzlich zu Mannitol. Cystein liegt bei pH 7 zwar als Zwitterion vor, wird erfindungsgemäß wegen seiner Elektroneutralität jedoch als nicht-ionisches Isotonisierungsmittel aufgefasst, welches praktisch nicht zur elektrischen Leitfähigkeit der Zusammensetzung beiträgt. Es wurde gefunden, dass Cystein bei pH 5,5 bis 7 keinerlei Puffereigenschaften besitzt.

Die Konzentration des Cysteins liegt bevorzugt im Bereich von 0,1±0,09 gl⁻¹, bevorzugter 0,1±0,08 gl⁻¹, noch bevorzugter 0,1±0,07 gl⁻¹, am bevorzugtesten 0,1±0,06 gl⁻¹, und insbesondere 0,1±0,05 gl⁻¹, bezogen auf die Zusammensetzung, vorzugsweise Infusionslösung.

Bevorzugt ist der Gewichtsanteil des Paracetamols größer als der Gewichtsanteil des Cysteins in der erfindungsgemäßen Zusammensetzung, vorzugsweise Infusionslösung. Vorzugsweise ist das relative Gewichtsverhältnis Paracetamol : Cystein > 50:1, bevorzugter > 60:1, noch bevorzugter > 70:1, am bevorzugtesten > 80:1 und insbesondere > 90:1 oder > 95:1.

In einer bevorzugten Ausführungsform enthält die erfindungsgemäße Zusammensetzung, vorzugsweise Infusionslösung, sowohl Mannitol als auch Cystein. Dabei ist der Gewichtsanteil des Mannitols bevorzugt größer als der Gewichtsanteil des Cysteins in der erfindungsgemäßen Zusammensetzung. Vorzugsweise ist das relative Gewichtsverhältnis Mannitol : Cystein > 100:1, bevorzugter > 200:1, noch bevorzugter > 250:1, am bevorzugtesten > 300:1 und insbesondere > 350:1 oder > 360:1.

In einer bevorzugten Ausführungsform enthält die erfindungsgemäße Zusammensetzung, vorzugsweise Infusionslösung, falls überhaupt, insgesamt höchstens 100 mmol/l Alkalimetallkationen, bevorzugter insgesamt höchstens 10 mmol/l, noch bevorzugter insgesamt höchstens 1.0 mmol/l, am bevorzugtesten insgesamt höchstens 0.1 mmol/l und insbesondere insgesamt höchstens 0.01 mmol/l. In einer bevorzugten Ausführungsform enthält die erfindungsgemäße Zusammensetzung praktisch kein Salz, weder gelöst noch fest. In diesem Zusammenhang werden zwitterionische Verbindungen unter isoelektrischen Bedingungen, z.B. Aminosäuren wie Cystein, nicht als Salze aufgefasst.

In einer bevorzugten Ausführungsform enthält die erfindungsgemäße Zusammensetzung, vorzugsweise Infusionslösung, keine Chelatbildner, z.B. EDTA.

In einer bevorzugten Ausführungsform enthält die erfindungsgemäße Zusammensetzung, vorzugsweise Infusionslösung, insgesamt höchstens 5 Inhaltsstoffe, d.h. neben Paracetamol und Wasser besteht die Zusammensetzung aus höchstens 3 weiteren Inhaltsstoffen. Ionische Verbindungen, welche in Wasser zu Kationen und Anionen dissoziieren, gelten dabei als 2 Verbindungen. Besonders bevorzugt enthält die erfindungsgemäße Zusammensetzung höchstens 4 Inhaltsstoffe. Besonders bevorzugt besteht die erfindungsgemäße Zusammensetzung aus Wasser, Paracetamol und Mannitol und/oder Cystein.

In einer besonders bevorzugten Ausführungsform enthält die erfindungsgemäße Zusammensetzung, vorzugsweise Infusionslösung, neben Wasser, Paracetamol und einem oder mehreren nicht-ionischen Isotonisierungsmitteln keinerlei weitere Inhaltstoffe.

Die erfindungsgemäße Zusammensetzung ist vorzugsweise gebrauchsfertig, d.h. sie kann bevorzugt sofort, insbesondere ohne bestimmte Vorbereitungsmaßnahmen verabreicht werden (einsatzbereit, *ready-to-use).* So ist es bevorzugt nicht erforderlich, dass die erfindungsgemäße Zusammensetzung verdünnt wird oder dass weitere Inhaltsstoffe zugegeben werden müssen, ehe die Zusammensetzung dem Patienten verabreicht werden kann.

Die erfindungsgemäße Zusammensetzung ist zur parenteralen Verabreichung vorgesehen, insbesondere zur intravenösen Infusion, d.h. es handelt sich bevorzugt um eine Infusionslösung. Zu diesem Zwecke ist es erforderlich, dass die Zusammensetzung eine physiologisch verträgliche Osmolarität (bzw. Osmolalität) aufweist. Vorzugsweise weist die erfindungsgemäße Zusammensetzung eine Osmolarität von wenigstens 0,22 osmol l⁻¹ auf, bevorzugter wenigstens 0,23 osmol l⁻¹, bevorzugter wenigstens 0,24 osmol l⁻¹, noch bevorzugter wenigstens 0,25 osmol l⁻¹, am bevorzugtesten wenigstens 0,26 osmol l⁻¹, und insbesondere wenigstens 0,27 osmol l⁻¹ auf. Vorzugsweise weist die erfindungsgemäße Zusammensetzung eine Osmolarität von höchstens 0,36 osmol l⁻¹ auf, bevorzugter höchstens 0,34 osmol l⁻¹, bevorzugter höchstens 0,32 osmol l⁻¹, noch bevorzugter höchstens 0,30 osmol l⁻¹, am bevorzugtesten höchstens 0,29 osmol l⁻¹, und insbesondere höchstens 0,28 osmol l⁻¹ auf. Im Vergleich enthält eine isotonische Kochsalzlösung 0,9% (Massenprozent) Natriumchlorid und entspricht mit einer Osmolarität von 308 mosmol/l annähernd der des Blutplasmas. Die theoretische Osmolarität einer Ringer-Infusionslösung liegt bei 309 mOsm/l. Die theoretische Osmolarität einer Ringer-Lactat-Lösung liegt zwischen 262 und 293 mOsm/l.

Die erfindungsgemäße Zusammensetzung, vorzugsweise Infusionslösung, zeichnet sich durch eine hervorragende Lagerstabilität aus. Es wurde überraschend gefunden, dass bei geringer elektrischer Leitfähigkeit und damit einhergehend entsprechend geringer Elektrolytkonzentration auf Puffersubstanzen völlig verzichtet werden kann und dennoch eine ausreichende Lagerstabilität erzielt wird. Bevorzugt beträgt der Gehalt an Paracetamol nach Lagerung bei 60 °C für 4 Wochen in geschlossenen Gefäßen mindestens 99,0% des ursprünglich, d.h. vor der Einlagerung, in der Zusammensetzung enthaltenen Paracetamols, bevorzugter mindestens 99,2%, noch bevorzugter mindestens 99,4%, am bevorzugtesten mindestens 99,6% und insbesondere mindestens 99,8%, vorzugsweise unter den im experimentellen Teil näher erläuterten Bedingungen.

Die erfindungsgemäße Zusammensetzung, vorzugsweise Infusionslösung, kann nach herkömmlichen, dem Fachmann bekannten Verfahren hergestellt werden. Vorzugsweise wird dabei zunächst
A) Wasser für Injektionszwecke mit einem Sauerstoffgehalt von weniger als 0,50 mg/l vorgelegt;
B) Paracetamol und die weitere Inhaltsstoffe in den gewünschten Mengen unter möglichst weitgehendem Ausschluss von Sauerstoff in der Vorlage A) gelöst ; und
C) gegebenenfalls der pH-Wert der Lösung durch Zugabe einer physiologisch verträglichen Säure bzw. Base auf den gewünschten Wert eingestellt.

Zweckmäßigerweise wird dann
D) die auf den gewünschten pH-Wert eingestellte Lösung durch einen Membranfilter 0,2 µm filtriert, anschließend in Behälter für Infusionslösungen abgefüllt und bei 121°C für 15 min hitzesterilisiert.

Eine weitere bevorzugte Variante des Verfahrens zur Herstellung der erfindungsgemäßen Lösung sieht vor, dass man zur Austreibung des Sauerstoffs ein inertes Gas durch das Wasser in Schritt A) leitet und dass man beim Vermischen in Schritt B) sowie gegebenenfalls bei allen weiteren Schritten unter Inertgasatmosphäre arbeitet.

Ein weiterer Aspekt der Erfindung betrifft Behälter, welche die erfindungsgemäße Zusammensetzung enthalten. Dabei liegt die erfindungsgemäße Zusammensetzung vorzugsweise als "Ready-to-use"-Präparat vor, d.h. kann sofort angewendet werden. Insbesondere sind vor der Anwendung bevorzugt keine Verdünnungs- oder Auflösungsschritte erforderlich.

Die erfindungsgemäße Zusammensetzung wird vorzugsweise in für Parenteralia üblichen Behältern verpackt. Die Behälter können Flaschen oder Beutel sein, wie sie für Injektionsfertiglösungen üblich sind. Behälter aus Glas oder Kunststoff sind bevorzugt. Sofern es sich um Kunststoffbehälter handelt, bestehen diese vorzugsweise aus einem auf Polyolefinen basierenden Material und werden ggfs. mit einem zweiten Beutel, der eine Sauerstoffbarriereschicht enthält, umgeben, evtl. mit einem Sauerstoffabsorber zwischen den Beuteln. Geeignete Verpackungsmaterialien sind dem Fachmann bekannt. In diesem Zusammenhang kann beispielsweise vollumfänglich verwiesen werden auf E. Bauer, Pharmaceutical Packaging Handbook, Informa Health Care 2009; oder D.A. Dean, Pharmaceutical Packaging Technology, Taylor & Francis 2000.

Die erfindungsgemäße Zusammensetzung kann unter Schutzgas verpackt sein, beispielsweise unter N₂, CO₂ oder Ar. In einer bevorzugten ausführungsform weist die erfindungsgemäße Zusammensetzung einen Gehalt an gelöstem Sauerstoff von höchstens 50 ppm, bevorzugter höchstens 20 ppm, noch bevorzugter höchstens 10 ppm, am bevorzugtesten höchstens 5 ppm und insbesondere höchstens 2 ppm oder höchstens 1 ppm auf.

Bevorzugt ist die Zusammensetzung frei von organischen Lösungsmitteln, weist einen pH-Wert im Bereich von 5,5 bis 7 und einen Sauerstoffgehalt von höchstens 2,00 mg/l auf, bevorzugter höchstens 1,50 mg/l, noch bevorzugter höchstens 1,25 mg/l, am bevorzugtesten höchstens 1,00 mg/l und insbesondere höchstens 0,50 mg/l.

Die parenterale Verabreichung der erfindungsgemäßen Zusammensetzung kann grundsätzlich auf allen üblichen Wegen erfolgen, insbesondere intravenös, intraarteriell, subcutan, intramuskulär, intraventrikulär, intracapsular, intraocular, intraspinal, intrazisternal, intraperitoneal, intranasal oder als Aerosol. Bevorzugt erfolgt die Verabreichung intravenös, wobei die Zusammensetzung bevorzugt als Infusionslösung vorliegt.

In einer bevorzugten Ausführungsform handelt es sich bei der erfindungsgemäßen Zusammensetzung um eine Infusionslösung, welche für die intravenöse Infusion über einen Zeitraum von 2 Minuten bis 24 Stunden hergerichtet ist, bevorzugter über einen Zeitraum von 3 Minuten bis 6 Stunden, noch bevorzugter 5 Minuten bis 1 Stunde, am bevorzugtesten 10 Minuten bis 45 Minuten und insbesondere 15 Minuten.

Ein weiterer Aspekt der Erfindung betrifft die vorstehend beschriebene Zusammensetzung, vorzugsweise Infusionslösung, zur Behandlung von Schmerz bzw. die Verwendung von Paracetamol zur Herstellung einer vorstehend beschriebenen Zusammensetzung zur Behandlung von Schmerz. Vorzugsweise handelt es sich bei dem Schmerz um mäßig starken Schmerz, bevorzugt um postoperativen Schmerz.

In einer bevorzugten Ausführungsform handelt es sich bei dem Patienten um geriatrische oder pädiatrische Patienten.

Die nachfolgenden Beispiele dienen zur Erläuterung der Erfindung, sind jedoch nicht einschränkend auszulegen:

Es wurden wässrige Lösungen von Paracetamol und weiteren Inhaltssoffen hergestellt. Die elektrische Leitfähigkeit der Lösungen wurde gemessen und die Lagerstabilität des Paracetamols wurde bestimmt anhand der Bildung von Abbauprodukten (zur Bildung des Dimers vgl. z.B. D.W. Pottert et al, J Biol Chem, 1985, 280(22), 12174-80; W. Clegg et al., Acta Crystallographica, 1998, C54, 1881-2).

Die Ergebnisse sind in den nachfolgenden beiden Tabellen zusammengefasst:

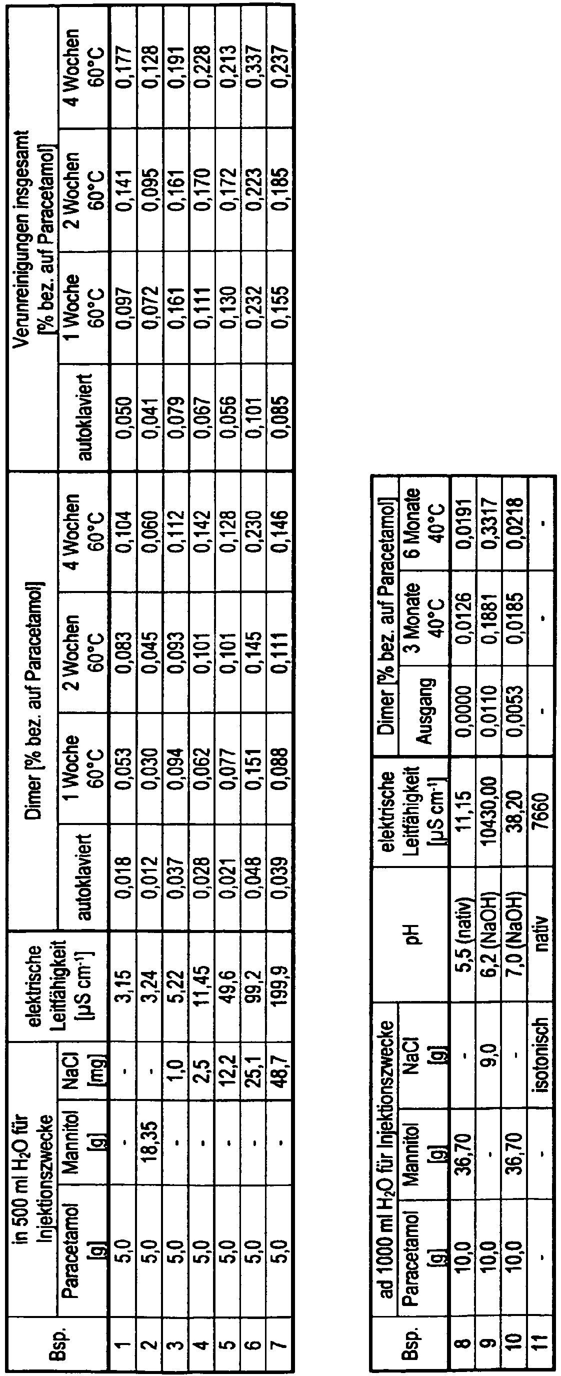

## Patentansprüche

1. Wässrige pharmazeutische Zusammensetzung in Form einer Infusionslösung, welche Paracetamol enthält und eine elektrische Leitfähigkeit von höchstens 200 µS cm⁻¹ aufweist, wobei die Zusammensetzung keine C₁-C₆-Alkanole und kein Polyethylenglykol enthält.

2. Zusammensetzung nach Anspruch 1, **dadurch gekennzeichnet, dass** sie eine Pufferkapazität β von höchstens 5,0 mmol l⁻¹ pH⁻¹ aufweist.

3. Zusammensetzung nach Anspruch 1 oder 2, **dadurch gekennzeichnet, dass** sie einen pH-Wert im Bereich von 5,0 bis 7,0 aufweist.

4. Zusammensetzung nach einem der vorstehenden Ansprüche, **dadurch gekennzeichnet, dass** sie ein nicht-ionisches Isotonisierungsmittel enthält.

5. Zusammensetzung nach Anspruch 4, **dadurch gekennzeichnet, dass** das nicht-ionische Isotonisierungsmittel ein Zuckeralkohol ist.

6. Zusammensetzung nach Anspruch 4 oder 5, **dadurch gekennzeichnet, dass** der Gewichtsanteil des nicht-ionischen Isotonisierungsmittels größer ist als der Gewichtsanteil des Paracetamols.

7. Zusammensetzung nach einem der vorstehenden Ansprüche, **dadurch gekennzeichnet, dass** sie eine Osmolarität von wenigstens 0,25 osmol l⁻¹ aufweist.

8. Zusammensetzung nach einem der vorstehenden Ansprüche, **dadurch gekennzeichnet, dass** sie praktisch kein Salz enthält.

9. Zusammensetzung nach einem der vorstehenden Ansprüche, **dadurch gekennzeichnet, dass** das Paracetamol in einer Konzentration von 10,0±5,0 g l⁻¹ vorliegt.

10. Zusammensetzung nach einem der vorstehenden Ansprüche, **dadurch gekennzeichnet, dass** der Gehalt an Paracetamol nach Lagerung bei 60 °C für 4 Wochen mindestens 99,0% des ursprünglich in der Zusammensetzung enthaltenen Paracetamols beträgt.

11. Zusammensetzung nach einem der vorstehenden Ansprüche, **dadurch gekennzeichnet, dass** sie gebrauchsfertig vorliegt.

12. Zusammensetzung nach einem der vorstehenden Ansprüche, **dadurch gekennzeichnet, dass** sie eine Osmolarität von höchstens 0,36 osmol l⁻¹ aufweist.

13. Zusammensetzung nach einem der vorstehenden Ansprüche, **dadurch gekennzeichnet, dass** die Infusionslösung für die intravenöse Infusion über einen Zeitraum von 2 Minuten bis 24 Stunden hergerichtet ist.

14. Zusammensetzung nach einem der Ansprüche 1 bis 13 zur Behandlung von Schmerz.

15. Zusammensetzung nach Anspruch 14, **dadurch gekennzeichnet, dass** der Schmerz postoperativer Schmerz ist.

## Claims

1. An aqueous pharmaceutical composition in the form of an infusion solution, which contains paracetamol and has an electrical conductivity of at most 200 µS cm⁻¹, wherein the composition does not contain any C₁-C₆-alkanols or any polyethylene glycol.

2. The composition according to claim 1, **characterized in that** it has a buffer capacity β of at most 5.0 mmol l⁻¹ pH⁻¹.

3. The composition according to claim 1 or 2, **characterized in that** it has a pH in the range from 5.0 to 7.0.

4. The composition according to one of the preceding claims, **characterized in that** it contains a nonionic isotonicizing agent.

5. The composition according to claim 4, **characterized in that** the nonionic isotonicizing agent is a sugar alcohol.

6. The composition according to claim 4 or 5, **characterized in that** the proportion by weight of the nonionic isotonicizing agent is greater than the proportion by weight of paracetamol.

7. The composition according to one of the preceding claims, **characterized in that** it has an osmolarity of at least 0.25 osmol/l⁻¹.

8. The composition according to one of the preceding claims, **characterized in that** it contains virtually no salt.

9. The composition according to one of the preceding claims, **characterized in that** the paracetamol is present in a concentration of 10.0 ± 5.0 g l⁻¹.

10. The composition according to one of the preceding claims, **characterized in that** the content of paracetamol after storage at 60°C for 4 weeks is at least 99.0% of the paracetamol originally contained in the composition.

11. The composition according to one of the preceding claims, **characterized in that** it is present in ready-to-use form.

12. The composition according to one of the preceding claims, **characterized in that** it has an osmolarity of at most 0.36 osmol/l⁻¹.

13. The composition according to one of the preceding claims, **characterized in that** the infusion solution for the intravenous infusion is prepared over a period of time of 2 minutes to 24 hours.

14. The composition according to one of Claims 1 to 13 for the treatment of pain.

15. The composition according to claim 14, **characterized in that** the pain is post-operative pain.

## Revendications

1. Composition pharmaceutique aqueuse sous forme d'une solution de perfusion qui contient du paracétamol et qui présente une conductibilité électrique d'au plus 200 *µ*S cm⁻¹, la composition ne contenant pas de C₁-C₆-alcanols, ni de polyéthylèneglycol.

2. Composition selon la revendication 1, **caractérisée en ce qu'**elle présente une capacité tampon ß d'au plus 5,0 mmoles l⁻¹ pH⁻¹.

3. Composition selon la revendication 1 ou 2, **caractérisée en ce qu'**elle présente un pH dans la plage de 5,0 à 7,0.

4. Composition selon l'une quelconque des revendications précédentes, **caractérisée en ce qu'**elle contient un agent d'isotonisation non ionique.

5. Composition selon la revendication 4, **caractérisée en ce que** l'agent d'isotonisation non ionique est un alcool de sucre.

6. Composition selon la revendication 4 ou 5, **caractérisée en ce que** la proportion pondérale de l'agent d'isotonisation non ionique est supérieure à la proportion pondérale du paracétamol.

7. Composition selon l'une quelconque des revendications précédentes, **caractérisée en ce qu'**elle présente une osmolarité d'au moins 0,25 osmole l⁻¹.

8. Composition selon l'une quelconque des revendications précédentes, **caractérisée en ce qu'**elle ne contient pratiquement pas de sel.

9. Composition selon l'une quelconque des revendications précédentes, **caractérisée en ce que** le paracétamol se trouve en une concentration de 10,0 ± 5,0 g l⁻¹_{.}

10. Composition selon l'une quelconque des revendications précédentes, **caractérisée en ce que** la teneur en paracétamol après l'entreposage à 60°C pendant 4 semaines vaut au moins 99,0% du paracétamol contenu initialement dans la composition.

11. Composition selon l'une quelconque des revendications précédentes, **caractérisée en ce qu'**elle se trouve sous forme prête à l'emploi.

12. Composition selon l'une quelconque des revendications précédentes, **caractérisée en ce qu'**elle présente une osmolarité d'au plus 0,36 osmole l⁻¹.

13. Composition selon l'une quelconque des revendications précédentes, **caractérisée en ce que** la solution de perfusion est préparée pour la perfusion par voie intraveineuse sur un laps de temps de 2 minutes à 24 heures.

14. Composition selon l'une quelconque des revendications 1 à 13 pour le traitement de la douleur.

15. Composition selon la revendication 14, **caractérisée en ce que** la douleur est la douleur postopératoire.
